(19) Europäisches Patentamt

European Patent Office

Office européen des brevets

(11) **EP 0 804 227 B1**

(12) **EUROPEAN PATENT SPECIFICATION**

(45) Date of publication and mention
of the grant of the patent:
**05.06.2002 Bulletin 2002/23**

(21) Application number: **96900321.9**

(22) Date of filing: **13.01.1996**

(51) Int Cl.[7]: **A61K 38/47**

(86) International application number:
**PCT/EP96/00135**

(87) International publication number:
**WO 96/21463 (18.07.1996 Gazette 1996/33)**

(54) **USE OF LYSOZYME DIMER FOR TREATING CANCER**

VERWENDUNG VON LYSOZYM-DIMER FÜR KREBSTHERAPIE

UTILISATION DE DIMERE DE LYSOZYME POUR TRAITER LE CANCER

(84) Designated Contracting States:
**AT BE CH DE DK ES FR GR IE IT LI LU MC NL PT SE**
Designated Extension States:
**LT LV SI**

(30) Priority: **13.01.1995 EP 95100446**
**07.07.1995 EP 95110638**

(43) Date of publication of application:
**05.11.1997 Bulletin 1997/45**

(60) Divisional application:
**00124590.1 / 1 086 703**

(73) Proprietor: **NIKA HEALTH PRODUCTS LIMITED**
**FL-9490 Vaduz (LI)**

(72) Inventor: **KICZKA, Witold**
**Princeton, NJ 08540 (US)**

(74) Representative: **Büchel, Kurt F., Dr. et al**
**Patentbüro Büchel & Partner AG**
**Letzanaweg 25-27**
**9495 Triesen (LI)**

(56) References cited:
EP-A- 0 181 634 WO-A-89/11294
WO-A-91/10731 WO-A-94/01127

• **EUROPEAN JOURNAL OF CANCER, vol. 15, no. 1, pages 85-91, XP002002104 BARTHOLEYNS ET AL: "IN VITRO AND IN VIVO ANTITUMOR EFFECT OF DIMERIZED RIBONUCLEASE A"** cited in the application
• **ARCHIVES INTERNATIONALES DE PHYSIOLOGIE ET DE BIOCHIMIE, vol. 87, pages 155-156, XP002002105 BARTHOLEYNS ET AL: "CYSTOSTATIC EFFECT AND INHIBITION OF PROTEIN DEGRADATION BY DIMERIZED LYSOZYME:COMPARISON WITH THE EFFECT OF DIMERIZED RIBONUCLEASE AND REVERSIBILITY BY GALACTOSE"**

**Description**

<u>TECHNICAL FIELD</u>

The present invention relates to new applications of lysozyme dimer and to compositions containing such dimer. The new applications are based on a common principle of non-specific stimulation of the immune system and are particularly useful for the prevention and/or treatment of symptoms or diseases in connection with an impaired function of the natural defensive and regenerative systems in the human and animal body.

<u>BACKGROUND OF THE INVENTION</u>

In the late eighties it was discovered that the dimerized forms of certain enzymes, while substantially retaining the beneficial properties of the corresponding monomers, turned to be by far less toxic than the monomers and in some instances did not even display negative side effects at all when used in therapeutic doses. Antiviral and antibacterial compositions comprising as the active ingredient lysozyme dimer or other dimerized enzymes have been described in WO 89/11294. It is reported therein that lysozyme dimer is capable of inhibiting proliferation of a number of bacterial strains cultivated on samples taken from patients, when applied in concentrations of 1.25 - 20 mg/ml of the culture. It is also reported that the dimer is effective in treating canine parvovirus (CPV) infections when administered orally twice a day at a dose of 1 - 2 mg/kg of body weight.
Later on, further attractive features of lysozyme dimers were found and additional therapeutical applications of the drug were developed, especially for the treatment of bacterial and viral infections as disclosed, for instance, in WO 94/01127.

Incidentally, this document also discloses the use of lysozyme dimers for treating AIDS - related kaposi sarcoma, insofar as it is a TNF$\alpha$ - induced disease. Treatment of inflammation as a symptom of cancer is also described, but not the treatment of cancer cell proliferation as such.

In WO 94/01127 a model theory is presented that can help to understand the different effects observed with the lysozyme dimer. Although the entire mode of action of the lysozyme dimer is not yet fully understood it appears that there is additional curative capability that cannot be explained by the bacteriolytic activity of the corresponding monomer. The inventors observed certain immunostimulative effects of the dimerized lysozyme, particularly concerning the modulation of cytokine levels. Moreover, from their experiments they concluded that lysozyme dimer seems to prevent the penetration of bacterial cells by viruses, presumably by blocking certain regions of the outer cell surface and probably comprising virus receptor proteins.

The prior art discloses further results obtained *in vitro* with lysozyme dimer. Particularly, Bartholeyns and Zenebergh (Europ. J. Cancer, Vol.15, 1979, 85-91) tested dimerized lysozyme for cytostatic activities against liver cancer cells (HCT) *in vitro.* They observed a 73% ± 15% inhibition of cancer cell multiplication in the cell culture (ibid., p.89, Table 2).

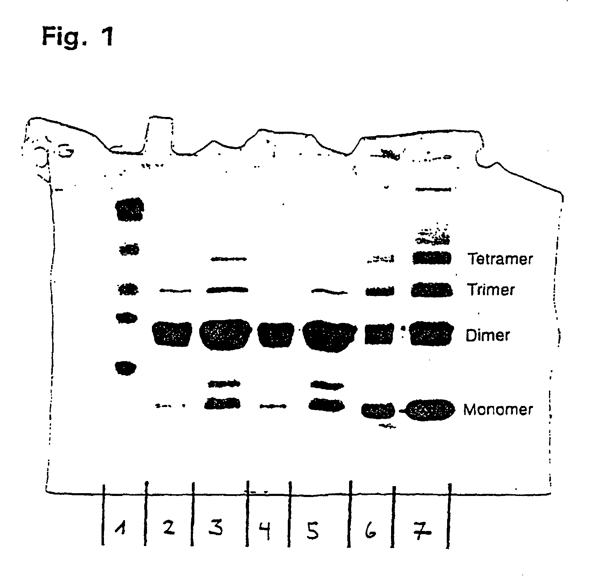

Surprisingly, except for WO 94/01127, no *in vivo* experiments with lysozyme dimer are reported so far. It is very strange and astonishing and up to now waits for explanation why neither Bartholeyns and Zenebergh nor any other researcher resumed this subject to promote further development of a promising discovery to combat cancer. A comparative showing (Fig. 1) of the purity of lysozyme dimer produced according to the method of Sorrentino et al., Eur. J. Biochem. 124, 183-189 (1982) and of the lysozyme dimer preferably used in the present invention revealed at least one possible reason: high concentrations of by-products such as lysozyme monomer, trimer and tetramer are found in the preparation produced according to Sorrentino et al., whereas the product preferably used in the present invention is highly purified, i.e., contains the desired lysozyme dimer in amounts of up to 90% by weight of the total lysozyme fraction of the preparation. A process for the manufacture of such highly purified lysozyme dimer has been described in WO 91/10731.

This strongly supports the assumption that the purity of the prior art lysozyme dimer was simply not good enough for *in vivo* experiments and applications because it was known in the art already 15 years ago that the monomeric form of lysozyme, despite its beneficial antibacterial activity, is rather toxic and can cause inflammations and severe allergies and even toxic shock symptoms.

In light of such circumstances it appears more understandable why no competent researcher including Bartholeyns and Zenebergh - although recommending lysozyme dimer as a promising candidate for further investigations - has carried out further experiments during the past ten to fifteen years to develop lysozyme dimer applications *in vivo*.

In spite of such lack of research activities of the scientific world possibly due to a prejudice of the art against the use of lysozyme dimer *in vivo,* the present inventors carried out further research and developmental work to improve the method of production and purification of the dimerized lysozyme and to find *in vivo* human and animal applications for the product which led, for instance, to the antiviral and antibacterial applications disclosed in WO 94/01127.

Moreover, based on their knowledge of low toxicity of dimerized lysozyme compared to the monomer and on the availability of a new, highly purified lysozyme dimer preparation the present inventors attempted and started anti-cancer trials with lysozyme dimer preparations *in vivo*, although the prior art did not suggest its use to treat diseases other than bacterial or viral infections. It is one of the biggest advantages of the present invention to provide for the use of a highly purified lysozyme dimer for the manufacture of a pharmaceutical composition to supplement or replace the extremely toxic anti-cancer drugs usually applied in conventional chemotherapy.

BRIEF DESCRIPTION OF THE DRAWINGS

Fig.1 displays a comparative showing of the purity of lysozyme dimer preparations manufactured according to two different prior art techniques.

DETAILED DESCRIPTION OF THE INVENTION

The highly purified lysozyme dimer referred to in the present invention can be used for the manufacture of pharmaceutical compositions directly applicable for the treatment of animals and humans due to its low amount of toxic monomer.

As indicated above, such purified lysozyme dimer contains about 10% wt. or less of unintended by-products, and can be obtained via dimerization of lysozyme monomers of any origin, e.g. of lysozyme monomers derived from humans, animals, eggs, plants, microorganisms, the monomers being either naturally isolated in native form or manufactured via chemical or genetic engineering methods to yield lysozyme monomers of the same or essentially the same chemical and biological nature as the naturally occurring ones.

In tests on *in vitro* cell cultures and *in vivo* test models, preferably mice and rat test models, the inventors of the present invention could successfully demonstrate that the applied lysozyme dimer compositions display remarkable potency in the inhibition or even total prevention of cell proliferation of cancerous tissues *in vitro* and *in vivo*. Such curative action could be confirmed for various types of cancer, including but not restricted to those that are known in the art to be induced or affected by viruses. These viruses comprise in particular the human papilloma virus, the Epstein-Barr virus, hepatitis B virus and retroviruses such as HTLV-I (adult T-cell leukaemia virus), HTLV-II (lymphoma virus) including subtypes.

The types of cancer induced or affected by the action of these viruses comprise i.e. cervix carcinoma, carcinoma of the urogenital tract, primary liver cell carcinoma, leukaemia, T-cell lymphoma, or any subtype thereof. It is an object of the present invention to provide for a pharmaceutical composition and a method to prevent and/or treat cancer in animals and humans.

The lysozyme dimer referred to herein can either directly be applied to the patients in need thereof or can be used for the manufacture of pharmaceutical compositions to be applied in usual galenic forms. Gels, ointments, or liquid compositions comprise the lysozyme dimer preferably in a concentration of about 0.01 - 10 mg/ml and frequently in a concentration of about 0.1-1.0 mg/ml. They are usually prepared as sterile and apyrogenic compositions and optionally further comprise at least one physiologically acceptable solvent and/or carrier and/or at least one suitable preservative.

The pharmaceutical compositions containing lysozyme dimer are useful and intended primarily for topical and/or parenteral application comprising local injection, e.g., in the vicinity of a solid tumor, or external applications on the surface of the body including subcutaneous injection. Intravenous injections may replace or additionally support topical applications in the course of a therapy. It has, however, also proven very efficient to administer lysozyme dimer compositions to mucosal membranes, preferably via inhalation (nasal, mouth, pharyngeal mucosa) of liquid compositions or topical application (e.g. vaginal, cervical mucosa) of liquid or creamy compositions or tampons impregnated with lysozyme dimer material.

In some cases it is preferred to apply the lysozyme dimer orally, preferably in usual galenic forms such as for instance tablets, capsules or dragees or in the form of pellets, granules, flocs or as a powder. These solid compositions frequently contain the active drug in an amount of about 0.01 - 10 mg, preferably about 0.01-1.0 mg per g of the total composition. It is also preferred that they further comprise at least one suitable carrier and/or preservative and/or other usual additives such as for instance a flavor or a colorant.

A composition for oral use may, however, also be in the form of an osmotic system. In certain cases such as, for instance, in order to prevent and/or treat cancer of the urogenital tract, the local application of antiseptic dressings or tampons impregnated with an effective dose of the highly purified lysozyme dimer can be useful and beneficial.

The various types of the above mentioned lysozyme dimer compositions are preferably administered in a single or repeatable dose of about 0.005 to 0.5 mg/kg of body weight, especially at a dose of about 0.01 to 0.1 mg/kg of body weight. It goes without saying that the required concentration of active lysozyme dimer in the final pharmaceutical composition depends primarily on the size of the human or animal patient and of the kind of therapy or prophylactic treatment scheduled for the concerned patient. In most cases, however, the above mentioned concentration ranges are sufficient for a proper treatment. Nevertheless, it might become necessary, particularly in veterinary medicine, to increase the actual lysozyme dimer concentrations in the composition to a value beyond 10 mg/ml and below the solubility product of the dimer in the respective solvent, i.e. to about 20 mg/ml of liquid or ointment. In doing so, the volume of the composition to be administered can be kept a reasonable minimum for the ease of handling.

If possible, a combined administration protocol of the aforementioned compositions is preferred over a single therapy of either oral, parenteral or topical application.

Due to the essentially untoxic character of the highly purified lysozyme dimer the compositions containing such dimer may be administered over a long period of time, i.e., months or even years without causing harmful side effects. The time intervals for prophylactic or therapeutic administration of the drug may typically range from one or more times daily to weekly and monthly dosages and may also comprise even longer intervals, depending on the respective patient and the urgency of treatment as well as on the efficacy of the immunostimulation by the lysozyme dimer.

As indicated above, the present invention was at least partly made possible through the availability of high grade lysozyme dimer. The striking difference of product quality and, in particular, of the undesired lysozyme monomer share, is demonstrated by Fig. 1:

Lane 1 represents LMW prestained protein standards:

Phosphorylase B 142.000 dalton, BSA 97.000, ovalbumin 50.000, carbonic anhydrase 35.100, soybean trypsin inhibitor 29.700, lysozyme 21.900 (Biorad, USA);

lanes 2 and 3 show purified lysozyme dimer LYDIUM KLP®602 (KLP-602), lot 506449, laboratory control; lane 2 loaded with 6.6 µg and lane 3 with 19.8 µg;
lanes 4 and 5 show another batch of KLP-602, lane 4 loaded with 6.6 µg and lane 5 with 19.8 µg;
lanes 6 and 7 show a lysozyme dimer preparation (KIW-607) manufactured according to Sorrentino et al., Eur. J. Bioch. 124, 183-189 (1982); lane 6 loaded with 6.6 µg and lane 7 with 19.8 µg.

The purified lysozyme dimer preparation KLP-602 contains four times more dimer than the compared product KIW-607, whereas the compared product KIW-607 contains six times more monomer than KLP-602.

In order that the invention described herein may be more fully understood, the following examples are set forth. It should be understood that these examples are for illustrative purposes only and are not to be construed as limiting this invention in any respect.

Example 1: Lysozyme dimer (KLP-602) in the treatment of lymphatic leukemia in AKR mice.

Materials and methods

Seven months old mice of both sexes of leukemic strain AKR, inoculated with Graffi virus from the Inbreeding Animal Center, Institute of Immunology and Experimental Therapy, Polish Academy of Sciences, Wroclaw, were used in the study. In AKR mice early activation of endogenous leukemic virus takes place, which leads to clinical manifestations of lymphatic leukemia at the age of seven to nine months. The mice were divided into three groups of 30 animals each:

Group I (experimental)

- animals were given KLP-602 subcutaneously once at a dose of 20 µg/kg in 0.3 cm$^3$ PBS;

Group II (experimental)

- animals received KLP-602 twice, at the same dose as above;

Group III (control)

- animals received 0.3 cm$^3$ PBS twice.

The mice were killed by bleeding three and six weeks after the last KLP-602 injection (10 mice from each group). Ten mice from each group were left alive.

The following examinations were performed:

1. Measurement of the total body weight and the mass of the internal organs such as liver, spleen, thymus and external cervical lymph nodes, with the estimation of the organ mass to total body weight ratio. The therapeutic activity ratio was evaluated using the formula L/K x 100 %, where L is the mean survival time of the mice treated with KLP-602 and K is the mean survival time of the controls.

2. Histopathological examinations of the biopsies of the liver, spleen, thymus and lymph nodes. Sections were stained with H+E of the Gomori method for reticular fibers.

3. The level of free radicals in the serum was estimated using the chemiluminescence (Chl) method which allows detection of very weak photon luminescence of the serum.

4. Phagocytic activity of the polymorphonuclears (PMN) was estimated using NBT and chemiluminescence (Chl) tests.

Results:

Single (Group I) and double (Group II) subcutaneous administration of KLP-602 to the highly leukemic AKR mouse strain; at a dose of 20 µg/kg, caused a statistically insignificant increase of body weight, and a decrease of the mass of internal organs such as: spleen, thymus, lymph nodes and liver (the last one was statistically insignificant) in comparison with the control animals (Group III) measured after three weeks of study (Table 1).

Increase of total body weight in both treated groups, decrease of the mass of the spleen, thymus and lymph nodes in Group I, and the increase of liver and thymus in Group II, when compared with the controls (Group III) occurred after six weeks of the study (Table 1).

Table 2 shows the ratio of the internal organs' weight to the total body weight. After three weeks the increase of thymus mass was observed in Group III (control) in comparison with both experimental groups (I and II). After six weeks, there was an increase of the mass of the spleen, thymus and lymph nodes in Groups II and III when compared with Group I.

The histopathological examinations conducted after three and six weeks revealed moderate (in the experimental groups) and excessive (in the control group) colonization of the cervical lymph nodes, thymus and spleen with lymphoid cells. However, the leukemic infiltrates in the liver were found only in the control. The lymphoid cells had oval or circular hyperchromatic nuclei with numerous nucleoli and atypical mitotic figures. These cells were accompanied by scanty low-differentiated centroblastic cells, reticulum cells and isles of proliferating megakaryocytes in the spleen. The stroma of the lymphatic organs such as the spleen, lymph nodes, and thymus contained scanty, thin reticulum fibers.

Chemiluminescence Examinations:

The activity of free radicals in the sera of the control mice was $K=18 \times 10^{-3}$ after three weeks of the study. In Group II the activity of free radicals was lower ($K=10 \times 10^{-3}$). After six weeks the free radicals level was $K=10 \times 10^{-3}$ in the control animals, lower in Group II ($K=16 \times 10^{-3}$), and slightly higher in Group I ($K=1.5 \times 10^{-3}$). The marked increase of the phagocytic activity of PMN was noted in both experimental groups as compared with the control group after three weeks and was: 4688 Chl (Group I), 3485 Chl (Group II), and only 2052 Chl for the control Group. After six weeks the phagocytic activity in Group II was equally high (3264 Chl) and it was lower in Group I (2477 Chl). A significant increase of phagocytic activity measured by a NBT test was noted after three weeks in both treated groups and was 0.27 and 0.26 in Groups I and II, respectively. After six weeks increase of phagocytic activity was lower and took place only in Group II. Phagocytic activity in the control group was 0.22.

The therapeutic efficacy index of KLP-602 was 128% and 150% in Groups I and II respectively, being above the minimal 125%. This indicates the marked immunostimulative properties of KLP-602 and qualifies it for further studies.

The leukemic infiltrates were considerably smaller in both experimental groups than in the control group and were even absent in some mice. The leukemic infiltrates were present in the thymus, spleen and lymph nodes. In addition, they were found in the liver of the control animals. The results of the study indicate that KLP-602 can partially inhibit or delay the progress of leukemia in AKR mice. To the contrary, the rapid enlargement of the lymphatic organs with morphological changes, typical for well differentiated lymphoid leukemia, occurred in the animals treated with the placebo.

Moreover, KLP-602 inhibits the increase of free radicals in mice serum. It inhibits free radical processes similar to the action of antioxidants such as vitamin E, C, or selenium. It is possible that the inhibition of the leukemic cell proliferation in AKR mice treated with KLP-602 depends on the inhibition of free radical induced processes. In control mice (without KLP-602) the level of free radicals was higher and correlated with the intensity of the disease.

KLP-602 also stimulates the phagocytic activity of the mononuclear phagocyte system (MPS) measured by two independent methods, NBT and Chl, and thus stimulates cell-mediated immunity. It can also modulate TNF synthesis. Moreover, the "tumor rejection antigens" present on the leukemic cell surface can be recognized by T-cells cooperating with MPS. The tumor anti-infiltrating lymphocytes (TIL), 50-100% more effective than lymphokine-activated killers (LAK), are among those T-cells. They have $CD_4$ or $CD_8$ or mixed phenotype and they express the IL-2 receptor on their surface. It is still unknown why they are paralyzed by neoplastic cells.

The reticulo-endothelial system with MPS cell stimulation is an essential immunological and antiviral mechanism. Stimulation of these cells results in interferon production and indirectly activates specific and non-specific immunological processes, B-cell, T-cell or NK dependent.

The MPS cells participate in immunological processes through their receptors such as Fc receptors, $C_3$ receptors or class II HLA receptors and also lectins, transferrins, urokinase, insulin and fifty other lesser-known receptors. Any changes of their expression and affinity indicate activation of the cells. MPS cells can phagocytize NO-sensitive cells because they can synthesise this substance. The activated phagocytes are called "angry macrophages" or "blood thirsty" cells. MPS cells can destroy viruses through their cytotoxic and cytolytic properties, defective virion production and interferon synthesis.

The promising results of the preliminary study on AKR mice indicate that KLP-602 can successfully be used in the treatment of leukemia in AKR mice.

Table 1.

| Total body mass (g) and mass of internal organs (mg) in control and experimental mice ($\bar{x} \pm S$; upper values indicate $\bar{x}$, lower values indicate S) | | | | | | |
|---|---|---|---|---|---|---|
| | Three Weeks | | | Six Weeks | | |
| Group | I | II | III | I | II | III |
| Total Body Mass | 25.27 | 26.20 | 23.96 | 26.77 | 27.30 | 25.57 |
| | 2.22 | 1.67 | 1.23 | 1.10 | 2.34 | 1.68 |
| Spleen | 0.07 | 0.08 | 0.09 | 0.07 | 0.23 | 0.22 |
| | 0.00 | 0.02 | 0.01 | 0.00 | 0.01 | 0.00 |
| Thymus | 0.06 | 0.07 | 0.08 | 0.08 | 0.21 | 0.15 |
| | 0.01 | 0.02 | 0.02 | 0.01 | 0.03 | 0.07 |
| Lymph Nodes | 0.04 | 0.04 | 0.05 | 0.04 | 0.07 | 0.08 |
| | 0.00 | 0.00 | 0.01 | 0.00 | 0.04 | 0.04 |
| Liver | 1.38 | 1.36 | 1.43 | 1.61 | 1.89 | 1.67 |
| | 0.15 | 0.38 | 0.05 | 0.18 | 0.42 | 0.15 |

Table 2.

| Internal organs' mass to total body mass ratio in control and experimental mice (%) | | | | | | |
|---|---|---|---|---|---|---|
| | Three Weeks | | | Six Weeks | | |
| Group | I | II | III | I | II | III |
| Spleen | 0.30 | 0.31 | 0.37 | 0.29 | 0.84 | 0.87 |
| Thymus | 0.23 | 0.26 | 0.33 | 0.33 | 1.06 | 0.61 |
| Lymph Nodes | 0.18 | 0.18 | 0.21 | 0.15 | 0.26 | 0.26 |
| Liver | 5.49 | 5.25 | 5.81 | 6.02 | 6.35 | 6.40 |

**Claims**

1. Use of a lysozyme dimer for the manufacture of a pharmaceutical composition for inhibiting or preventing cell proliferation of cancerous tissues other than Kaposi's sarcoma.

**2.** Use according to claim 1, for preventing or treating a cancer induced or affected by a virus.

**3.** Use according to claim 2, wherein the virus is selected from the group consisting of human papilloma virus, Epstein-Barr virus, hepatitis B virus, HTLV-I (adult T-cell leukaemia virus), HTLV-II (lymphoma virus) and HIV-1 (human immunodeficiency virus).

**4.** Use according to any one of claims 1 to 3, wherein the cancer is selected from the group consisting of cervix carcinoma, carcinoma of the urogenital tract, primary liver cell carcinoma, leukemia, and T-cell lymphoma.

**5.** Use according to claim 4, wherein said leukemia is lymphatic leukemia.

**6.** Use according to any one of claims 1 to 5, wherein said treatment comprises inhibiting free radical processes in serum.

**7.** Use according to any one of claims 1 to 4, wherein said lysozyme dimer contains about 10% by weight or less of unintended by-products and is essentially free from a monomeric form of lysozyme.

**8.** Use according to any one of the preceding claims, wherein the pharmaceutical composition is administrable in a single or repeatable dose of 0.005 to 0.5 mg/kg body weight, preferably 0.01 to 0.1 mg/kg body weight, of a human or animal individual.

**9.** Use according to any one of the preceding claims, wherein the pharmaceutical composition is in the form of a gel, an ointment or a liquid composition and comprises said lysozyme dimer in an amount of from 0.01 to 10 mg/ml, preferably 0.01 to 1 mg/ml.

**10.** Use according to any one of claims 1 to 8, wherein the pharmaceutical composition is in a solid form suitable for oral administration and comprises said lysozyme dimer in an amount of from 0.01 to 10 mg/g, preferably 0.01 to 1 mg/g.

**Patentansprüche**

**1.** Verwendung eines Lysozym-Dimers für die Herstellung einer pharmazeutischen Zusammensetzung zur Hemmung oder Verhütung der Zellenproliferation von Krebsgeweben ausser denen von Morbus-Kaposi.

**2.** Verwendung nach Anspruch 1 zur Verhütung oder Behandlung eines durch einen Virus induzierten oder beeinflussten Krebses.

**3.** Verwendung nach Anspruch 2, worin der Virus aus der Gruppe ausgewählt ist, die aus menschlichem Papillomvirus, Epstein-Barr-Virus, Hepatitis-B-Virus, HTLV-I (adulten T-Zellen-Leukämievirus), HTLV-II (Lymphomvirus) und HIV-1 (menschlichem Immunschwächevirus) besteht.

**4.** Verwendung nach einem der Ansprüche 1 bis 3, worin der Krebs aus der Gruppe ausgewählt ist, die aus Cervix-Carcinom, Carcinom der Urogenitalwege, primärem Leberzellencarcinom und T-Zellen-Lymphom besteht.

**5.** Verwendung nach Anspruch 4, worin die benannte Leukämie lymphatische Leukämie ist.

**6.** Verwendung nach einem beliebigen der Ansprüche 1 bis 5, worin die benannte Behandlung umfasst, Freie-Radikal-Prozesse im Serum zu hemmen.

**7.** Verwendung nach einem beliebigen der Ansprüche 1 bis 4, worin das benannte Lysozym-Dimer etwa 10 Gewichtsprozent oder weniger an unbeabsichtigten Nebenprodukten enthält und im Wesentlichen frei von einer monomeren Form von Lysozym ist.

**8.** Verwendung nach einem beliebigen der vorstehenden Ansprüche, worin die pharmazeutische Zusammensetzung in einer einzelnen oder wiederholbaren Dosis von 0,005 bis 0,5 mg/kg Körpergewicht, vorzugsweise von 0,01 bis 0,1 mg/kg Körpergewicht eines menschlichen oder tierischen Individuums verabreicht werden kann.

**9.** Verwendung nach einem beliebigen der vorstehenden Ansprüche, worin die pharmazeutische Zusammensetzung in Gestalt eines Gels, einer Salbe oder einer flüssigen Zusammensetzung vorliegt und das benannte Lysozym-Dimer in einer Menge von 0,01 bis 10 mg/ml, vorzugsweise von 0,01 bis 1 mg/ml enthält.

**10.** Verwendung nach einem beliebigen der Ansprüche 1 bis 8, worin die pharmazeutische Zusammensetzung in fester Gestalt vorliegt, die für eine orale Verabreichung geeignet ist und das benannte Lysozym-Dimer in einer Menge von 0,01 bis 10 mg/g, vorzugsweise von 0,01 bis 1 mg/g enthält.

**Revendications**

**1.** Utilisation d'un dimère de lysozyme en vue de la fabrication d'une composition pharmaceutique destinée à empêcher ou prévenir une prolifération de cellules de tissus cancéreux autres que la maladie de Kaposi.

**2.** Utilisation selon la revendication 1, en vue de prévenir ou de traiter un cancer induit ou provoqué par un virus.

**3.** Utilisation selon la revendication 2, dans laquelle le virus est choisi parmi le groupe constitué du virus du papillome humain, du virus Epstein-Barr, du virus de l'hépatite B, de HTLV-I (virus de la leucémie de lymphocytes T chez l'adulte), de HTLV-II (virus du lymphome) et de VIH-1 (virus de l'immunodéficience humaine).

**4.** Utilisation selon l'une quelconque des revendications 1 à 3, dans laquelle le cancer est choisi parmi le groupe constitué d'un cancer du col, d'un cancer des voies uro-génitales, d'un cancer primitif des cellules hépatiques, d'une leucémie, et d'un lymphome des lymphocytes T.

**5.** Utilisation selon la revendication 4, dans laquelle ladite leucémie est une leucémie lymphatique.

**6.** Utilisation selon l'une quelconque des revendications 1 à 5, dans laquelle ledit traitement comprend l'empêchement des processus à radicaux libres dans le sérum.

**7.** Utilisation selon l'une quelconque des revendications 1 à 4, dans laquelle ledit dimère de lysozyme contient environ 10 % en poids ou moins de sous-produits non prévus et est essentiellement dépourvu d'une forme monomère de lysozyme.

**8.** Utilisation selon l'une quelconque des revendications précédentes, dans laquelle la composition pharmaceutique peut être administrée sous forme d'une dose unique ou d'une dose répétable de 0,005 à 0,5 mg/kg de masse corporelle, de préférence de 0,01 à 0.1 mg/kg de masse corporelle, d'un sujet humain ou animal.

**9.** Utilisation selon l'une quelconque des revendications précédentes, dans laquelle la composition pharmaceutique est sous la forme d'un gel, d'une pommade ou d'une composition liquide et comprend ledit dimère de lysozyme dans une proportion de 0,01 à 10 mg/ml, de préférence de 0,01 à 1 mg/ml.

**10.** Utilisation selon l'une quelconque des revendications 1 à 8, dans laquelle la composition pharmaceutique est sous une forme solide convenant à une administration par voie orale et comprend ledit dimère de lysozyme dans une proportion de 0,01 à 10 mg/g, de préférence de 0,01 à 1 mg/g.

## Fig. 1

Tetramer

Trimer

Dimer

Monomer

1 | 2 | 3 | 4 | 5 | 6 | 7